# DEMANDE DE BREVET EUROPEEN

(11) **EP 1 795 174 A1**
(43) Date de publication de la demande: **13.06.2007**
(21) Numéro de dépôt: 06124928.0
(22) Date de dépôt: 28.11.2006
(51) Int. Cl.: A61K 8/02, A61K 8/73, A61K 8/81

(54) **Patch soluble**

(30) Priorité: 07.12.2005 FR 0553752
(71) Demandeur: L'Oreal, 75008 Paris (FR)
(72) Inventeur: Aubrun-Sonneville, Odile, 92160 Antony (FR)
(74) Mandataire: Rasson, Catherine

(57) **Abrégé**

La présente invention porte sur un article cosmétique ou dermatologique comportant :
- un support sous forme d'au moins une nappe de fibres solubles dans l'eau à une température inférieure ou égale à 30°C, et
- au moins une composition portée sur le support et contenant au moins un gélifiant hydrosoluble qui gonfle en moins de 30 secondes dans l'eau à une température de 20°C à 30°C.

L'article selon l'invention constitue notamment un patch.

## Description

La présente invention concerne des patchs comprenant un support soluble dans l'eau et une composition portée par le support, et leurs utilisations notamment dans le domaine cosmétique.

Dans le domaine cosmétique, il est connu d'utiliser des patchs ou timbres cosmétiques qui sont constitués d'un support insoluble dans l'eau, imprégné d'une composition cosmétique. Un patch est par définition appliqué sur une zone limitée de peau, zone plus ou moins grande, ce qui permet la libération lente d'une substance active par transdermie, et ainsi le traitement efficace de zones spécifiques. Toutefois, les patchs sur support insoluble présentent l'inconvénient d'une part de générer des déchets à éliminer, et d'autre part de ne pouvoir remplir qu'une seule fonction et de n'être pas modulable en fonction du but recherché, contribuant à l'augmentation du nombre de produits à utiliser dans une routine de soin. Or, il peut être intéressant de disposer de produits pouvant présenter deux fonctions selon le mode d'application, par exemple un nettoyant moussant qui peut être utilisé quotidiennement mais qui peut aussi être utilisé de façon hebdomadaire comme traitement spécifique avec un temps de pose pour une plus grande efficacité.

Par ailleurs, il a été décrit des films fins hydrosolubles contenant des actifs, films qui sont humectés avec de l'eau pour donner une composition (solution, dispersion ou émulsion) qui est ensuite étalée sur la peau. Le document WO-A-02/05789 décrit par exemple de tels films. Toutefois, ces films présentent l'inconvénient d'être de fabrication complexe avec mise en solution des composants, chauffage et séchage pour obtenir un film sec. En outre, ils sont difficiles à sécher si l'épaisseur est trop grande, et ils sont fragiles et difficiles à manipuler si la taille est trop grande.

Il subsiste donc le besoin de disposer de patchs n'ayant pas les inconvénients de ceux de l'art antérieur, et notamment de disposer de patchs à rincer ou à essuyer, constituant des articles pouvant avoir plusieurs fonctions, par exemple utilisables selon les besoins des utilisateurs comme patch ou produit de soin, pouvant constituer par exemple un produit quotidien à utiliser dilué ou un produit hebdomadaire à utiliser sur une zone définie avec un temps de pose.

La présente demande répond à ce besoin. En effet, la demanderesse a trouvé de manière surprenante qu'il était possible de préparer des articles comportant un support contenant des fibres solubles et un gélifiant particulier, et d'obtenir par dissolution de ces articles dans l'eau ou dans un milieu aqueux, une composition gélifiée, notamment une composition cosmétique gélifiée ayant de bonnes propriétés cosmétiques et pouvant constituer un patch. Cet article se différencie du film décrit dans le document WO-A-02/05789 à la fois par le support qui n'est pas un film soluble mais un non-tissé de fibres solubles, et par le géifiant porté par le support qui doit avoir une propriété particulière de gonflement, ce qui permet d'obtenir un patch facilement soluble et efficace.

L'utilisation de ce type d'articles permet de lever les contraintes de formulation dans la mesure où ces articles peuvent conduire à une large gamme de produits, des gels aux crèmes, pour différentes applications, en fonction de la composition appliquée sur le support.

Ainsi, l'invention réside, selon l'un de ses aspects, dans un article cosmétique ou dermatologique comportant :
- un support sous forme d'au moins une nappe comprenant des fibres solubles dans l'eau à une température inférieure ou égale à 30°C, et
- une composition portée par le support, contenant au moins un gélifiant hydrosoluble qui gonfle en moins de 30 secondes dans l'eau à une température de 20 °C à 30 °C.
   On entend par « article cosmétique ou dermatologique», un produit cosmétique ou dermatologique comprenant un support solide comportant une composition portée par ce support. Cet article peut constituer notamment un patch à humidifier et appliquer sur la peau.
   On entend dans la présente demande par « portée par le support », le fait que la composition peut être soit mise sur le support soit introduite dans la cavité formée par le support quand celui-ci comporte au moins deux nappes formant une cavité.
   On entend par « température inférieure ou égale à 30°C », une température ne dépassant pas 30°C mais n'étant pas inférieure à 0°C, par exemple allant de plus de 0°C à 30°C, mieux de 5°C à 30°C et encore mieux de 10°C à 30°C.
   On entend par « gélifiant qui gonfle en moins de 30 secondes dans l'eau à une température de 20°C à 30°C », un gélifiant qui gonfle rapidement après introduction dans l'eau en une concentration de 5 à 50 % en poids, cette concentration dépendant du gélifiant pour donner sans agitation un gel visqueux qui ne coule pas. Un tel gélifiant est apte à son état sec à absorber spontanément au moins 10 fois son poids d'eau, notamment d'eau distillée, « spontanément » signifiant sans agitation et sans chauffage, c'est-à-dire à la température ambiante (20 à 25 °C).
   Lors de l'utilisation, le ou les gélifiants utilisés selon l'invention vont gonfler rapidement dans l'eau pour former un matériau qui ne coule pas et qui peut ainsi être appliqué facilement sur la zone à traiter.
   La présence du gélifiant hydrosoluble permet la transformation de l'article en gel ou pâte plus ou moins lisse, après un temps de contact inférieur à 30 minutes avec une faible quantité d'eau à température ambiante (20° à 30°C), de façon à pouvoir être utilisé en particulier comme patch à rincer à appliquer localement (par exemple sur la zone du nez ou du front) avec un temps de pose pour une action plus forte, ou comme produit à appliquer sur une zone plus étendue (par exemple tout le visage). article multifonction selon les besoins de l'utilisateur. On entend par « article multifonction », un article qui, selon le besoin de l'utilisateur, peut être employé de manière différente, par exemple comme patch dans une zone limitée ou comme produit de nettoyage dans une zone plus large. Dans le cas du patch, l'article est simplement humidifié de façon à obtenir un gel épais. Dans le cas du produit de nettoyage, l'article peut-être solubilisé dans une plus grande quantité d'eau de manière à obtenir un produit plus facile à étaler et moins concentré en actif pour traiter une zone étendue.
   Par exemple, l'article peut constituer un patch pour peau grasse pour le nez, à utiliser une fois par semaine, permettant un traitement ciblé avec des actifs kératolytiques et/ou des anti-bactériens ; le patch est humidifié avant emploi, on le laisse poser quelques minutes (par exemple 10 minutes), puis on le rince et/ou on l'essuie ensuite. Avant rinçage ou essuyage, il peut également être étalé sur le reste du visage en ajoutant ou non de l'eau, de manière à traiter ou nettoyer l'ensemble du visage avec une formule moins concentrée.
   L'article selon l'invention peut également constituer une formule à réhydrater avant emploi avec une quantité d'eau à ajuster par le consommateur en fonction de la texture ou de l'activité souhaitées.
   De manière préférée, cet article constitue un patch soluble ou partiellement soluble. « Partiellement soluble » signifie que l'article comporte un support comportant au moins 60 % en poids de fibres solubles dans l'eau par rapport au poids total de fibres.
   Les termes « nappe » et « couche » doivent être considérés comme synonymes dans la présente demande. Le support de la présente invention se présente sous forme d'une ou plusieurs nappes de fibres, ce qui est différent des films fins hydrosolubles qui ne sont pas sous forme de nappes de fibres. Par rapport à ces films fins hydrosolubles, les supports à base de nappes de fibres hydrosolubles selon l'invention présentent l'avantage de permettre l'incorporation de constituants incompatibles, d'être plus simples à mettre oeuvre car ils ne nécessitent pas de prémélange ni de mise en solution des composants, ni de chauffage pour évaporer le solvant, le procédé étant également plus rapide et moins coûteux. En outre, les supports selon l'invention ont l'avantage de permettre une plus grande diversité dans le choix de la forme et de l'aspect de l'article car la nappe de fibres peut avoir une épaisseur et une densité variables donnant accès à une grande variété de forme et de taille, alors que le film fin est difficile à sécher si l'épaisseur est trop grande, et il est fragile et difficile à manipuler si la taille est trop grande.
   Selon un mode préféré de réalisation de l'invention, l'article se présente sous la forme d'au moins deux nappes définissant entre elles une cavité, l'une au moins des nappes comprenant des fibres solubles dans l'eau à une température inférieure ou égale à 30°C,
- la cavité contenant une composition comportant un gélifiant hydrosoluble qui gonfle en moins de 30 secondes dans l'eau à une température de 20°C à 30°C.

Les deux nappes sont assemblées à leur périphérie et forment ainsi une cavité permettant d'introduire la composition contenant. le gélifiant hydrosoluble.

Les nappes peuvent être formées entièrement de fibres solubles dans l'eau ou bien l'une des nappes peut être constituée entièrement de fibres solubles et l'autre nappe peut être constituée de fibres insolubles ou à la fois de fibres solubles et de fibres insolubles dans l'eau, ou bien les deux nappes peuvent être constituées à la fois de fibres solubles et de fibres insolubles,

Selon un mode particulier de réalisation de l'invention, au moins une des nappes est constituée exclusivement de fibres solubles dans l'eau.

Par humidification ou dissolution de l'article selon l'invention dans l'eau ou une composition aqueuse, on obtient une composition gélifiée pour application topique, notamment cosmétique ou dermatologique.

Ainsi, l'invention a encore pour objet, selon un autre de ses aspects, une composition pour application topique, obtenue par la dissolution dans l'eau, d'un article tel que défini ci-dessus, c'est-à-dire une composition obtenue par dissolution d'un support sous forme d'au moins une nappe comprenant des fibres solubles dans l'eau à une température inférieure ou égale à 30°C, le dit support portant au moins un gélifiant hydrosoluble qui gonfle en moins de 30 secondes dans l'eau à une température de 20°C à 30°C. Cette composition peut être obtenue à partir d'un support comprenant une ou plusieurs couches de fibres. La température de dissolution de l'article dans l'eau est généralement la température ambiante (20 à 30°C) mais peut être supérieure à la température ambiante si l'on le souhaite selon l'utilisation envisagée.

L'invention a encore pour objet, selon un autre de ses aspects, un procédé de traitement cosmétique des matières kératiniques telles que la peau, les cheveux, les muqueuses et les phanères, et notamment pour le traitement de la peau, comportant :
- la formation d'une composition cosmétique par dissolution dans l'eau, d'un support comportant au moins une nappe comprenant des fibres solubles dans l'eau à une température inférieure ou égale à 30°C , et portant au moins un gélifiant hydrosoluble qui gonfle en moins de 30 secondes dans l'eau à une température de 20°C à 30°C,
- l'application de la composition ainsi formée sur les matières kératiniques.

Le traitement cosmétique peut être notamment le soin de la peau.

Par «soluble dans l'eau à une température inférieure ou égale à 30°C », il faut comprendre une solubilisation dans l'eau à une température allant jusqu'à 30°C avec l'aide d'une agitation manuelle et/ou d'une friction du support le cas échéant, dans un laps de temps typiquement inférieur à 5 mn, de préférence inférieur à 1 mn, de préférence inférieur à 30 secondes. L'invention n'exclut pas qu'une eau de température supérieure à 30 °C soit utilisée pour dissoudre le support.

L'article selon l'invention étant destinée à une application topique, comprend un milieu physiologiquement acceptable. On entend par « milieu physiologiquement acceptable » un milieu compatible avec les matières kératiniques telles que la peau, les lèvres, les ongles, le cuir chevelu et/ou les cheveux. Il en est de même du support, ainsi que de la composition portée par le support.

L'article selon l'invention ne contient pas d'adhésif, mais il peut adhérer à la peau quand il est humidifié.

Cet article est flexible, c'est-à-dire souple. Par «souple», il faut comprendre un article pouvant être comprimé ou pouvant fléchir sans se rompre, capable de s'adapter aux reliefs du corps humain. Un article souple réalisé sous la forme d'une nappe fibreuse peut dans certains exemples de réalisation être replié sur lui-même au moins une fois sans se casser en deux morceaux.

Cet article est généralement à usage unique.

Par ailleurs, l'article est généralement sec au toucher avant l'utilisation.

Après sa fabrication, l'article peut être par exemple conditionné en vrac dans une boîte ou dans un emballage individuel. Le cas échéant, les articles sont conditionnés en chapelet. Les articles peuvent encore être repliés sur eux-mêmes et intercalés, de telle sorte que le retrait d'un article amène le suivant dans une configuration facilitant sa préhension.

Ainsi, l'invention a encore pour objet, selon un autre de ses aspects, un ensemble comportant :
- un emballage,
- au moins un article tel que défini plus haut.

L'invention offre ainsi de nouvelles possibilités pour le conditionnement et la formulation des patchs.

Dans un exemple de mise en oeuvre de l'invention, l'article formé par le support et la composition contenant le gélifiant hydrosoluble, est destiné à être mis en contact avec l'eau avant son utilisation. Le support est ainsi d'abord solubilisé entièrement avant que l'article ne soit appliqué sur le corps humain. Selon la quantité d'eau rajoutée à l'article pour solubiliser le support, on peut aisément ajuster la viscosité apparente du patch obtenu.

Dans une autre variante de mise en oeuvre de l'invention, l'article formé par le support et la composition portée par le support, est mis au contact d'une région du corps humain, par exemple la peau ou les cheveux, avant sa solubilisation complète, voire avant d'être mouillé. Cela peut permettre par exemple, selon la quantité d'eau ajoutée, de modifier les propriétés en fonction du résultat souhaité. L'eau peut être versée sur l'article alors que celui-ci n'est pas au contact de la région du corps à traiter, ou bien la région du corps peut encore être mouillée, ou encore de l'eau peut être projetée ou versée sur le support alors que l'article est au contact de la région à traiter.

Selon un mode préféré d'utilisation de l'article selon l'invention, on passe l'article sous l'eau et on l'applique sur la zone à traiter.

### Support

Le support se présente sous forme d'une nappe comprenant des fibres hydrosolubles, c'est-à-dire des fibres solubles dans l'eau à une température inférieure ou égale à 30°C, de préférence solubles dans l'eau à une température inférieure ou égale à 20°C, c'est-à-dire ayant une température de dissolution dans l'eau allant de plus de 0°C à 30°C, de préférence de plus 0°C à 20°C, et par exemple de 5 °C à 30 °C, et mieux de 5 à 20 °C.

Le support peut avoir toute forme appropriée pour l'utilisation visée, par exemple une forme rectangulaire, ronde ou ovale, et il a de préférence des dimensions permettant sa préhension entre deux doigts au moins. Ainsi, le support peut avoir par exemple une forme ovoïde d'environ 2 à 10 cm de long et d'environ 0,5 à 4 cm de large, ou une forme de disque d'environ 2 à 10 cm de diamètre, ou une forme de carré d'environ 5 à 15 cm de côté, ou une forme de rectangle d'une longueur d'environ 5 à 15 cm, étant entendu qu'il peut avoir toute autre forme et dimension appropriées pour l'utilisation recherchée.

Les fibres du support sont généralement enchevêtrées pour former la nappe de fibres. Comme indiqué plus haut, on entend par « nappe comprenant des fibres solubles dans l'eau », une nappe pouvant être entièrement constituée de fibres solubles dans l'eau ou une nappe pouvant comporter à la fois des fibres solubles dans l'eau et des fibres insolubles dans l'eau, les fibres solubles devant être en plus grande quantité que les fibres insolubles. La nappe de fibres doit comporter au moins 60 % en poids de fibres solubles, de préférence au moins 70 % et mieux au moins 80 % en poids par rapport au poids total des fibres. Elle peut ainsi comporter, par exemple, plus de 95 % en poids, voire plus de 99 % en poids et même 100 % en poids de fibres hydrosolubles par rapport au poids total des fibres du support. Ainsi, le support peut être constitué entièrement de nappes de fibres solubles ou il peut être constituée de nappes comportant un mélange de fibres solubles et de fibres insolubles, les fibres insolubles étant selon la définition de la présente invention, des fibres qui ne sont pas solubles dans l'eau à une température inférieure ou égale à 30°C. Le fait d'avoir des fibres insolubles peut permettre d'avoir un produit à effet thermique qui soit en même temps un produit de gommage doux (scrub), les fibres insolubles constituant le composé exfoliant.

Ainsi, le support peut être formé de deux nappes constituées de fibres solubles dans l'eau, ou encore d'une nappe constituée de fibres solubles dans l'eau et d'une nappe comprenant à la fois de fibres solubles et de fibres insolubles, ou bien aussi d'une nappe constituée de fibres solubles dans l'eau et d'une nappe constituée de fibres insolubles dans l'eau, ou bien de deux nappes comprenant à la fois de fibres solubles et de fibres insolubles. Il peut y avoir aussi plus de deux nappes.

Selon un mode préféré de réalisation de l'invention, le support est dépourvu de fibres insolubles dans l'eau et il est composé uniquement de fibres solubles de l'eau, de sorte qu'il soit entièrement soluble dans l'eau.

Les fibres solubles peuvent être en tout matériau soluble susceptible d'étre filé en fibres. De préférence, les fibres solubles dans l'eau sont réalisées avec de l'alcool polyvinylique (PVA) selon un procédé qui leur confère la solubilité recherchée, le PVA pouvant avoir plusieurs grades de polymérisation.

Des fibres de PVA solubles dans l'eau à une température inférieure ou égale à 30°C sont commercialisées par la société japonaise KURARAY sous la dénomination commerciale KURALON K-II WN2. Le procédé de fabrication de ces fibres comporte la préparation d'une solution à filer par dissolution d'un polymère à base de PVA soluble dans l'eau, dans un premier solvant organique, le filage de la solution dans un second solvant organique pour obtenir des filaments solidifiés et l'étirage humide des filaments dont on enlève le premier solvant puis que l'on sèche et que l'on soumet à un traitement thermique. La section de ces fibres peut être sensiblement circulaire. Ces fibres ont une résistance à la traction d'au moins 2,7 g/dtex (3 g/d). La demande EP-A-0 636 716 décrit de telles fibres hydrosolubles à base de PVA et leur procédé de fabrication.

L'invention n'est pas limitée à l'emploi de PVA, et on peut utiliser aussi des fibres réalisées dans d'autres matériaux hydrosolubles sous réserve que ces matériaux se dissolvent dans de l'eau ayant la température recherchée, par exemple des fibres de polysaccharides commercialisées sous la dénomination LYSORB par la société LYSAC TECHNOLOGIES, INC ou des fibres à base de polymères polyholosides comme le glucomannane ou l'amidon.

La nappe de fibres peut comporter, le cas échéant, un mélange de différentes fibres solubles dans l'eau à des températures différentes (jusqu'à 30°C).

Les fibres peuvent être composites, et elles peuvent comporter par exemple un coeur et une gaine n'ayant pas la même nature, par exemple formés de différents grades de PVA.

Quand la nappe de fibres contient des fibres insolubles, celles-ci peuvent être en toute matière habituellement utilisée comme fibres insolubles ; ce peut être par exemple des fibres de soie, de coton, de laine, de lin, de cellulose extraites notamment du bois, des légumes ou des algues, de polyamide (Nylon^{®}), d'acide polylactique, de cellulose modifiée (rayonne, viscose, acétate notamment d'acétate de rayonne), de poly-p-phénylène téréphtalamide notamment de Kevlar^{®}, en acrylique notamment de polyméthacrylate de méthyle ou de poly 2-hydroxyéthyl méthacrylate, de polyoléfine et notamment de polyéthylène ou de polypropylène, de verre, de silice, d'aramide, de carbone notamment sous forme graphite, de Téflon^{®}, de collagène insoluble, de polyesters, de polychlorure de vinyle ou de vinylidène, d'alcool polyvinylique, de polyacrylonitrile, de chitosane, de polyuréthane, de polyéthylène téréphtalate, de fibres formées d'un mélange des composés mentionnés ci-dessus, comme des fibres de polyamide/polyester ou de viscose/polester. Les non tissés sont décrits de façon générale dans RIEDEL «Nonwoven Bonding Methods & Materials», Nonwoven World (1987), incorporé ici par référence.

Dans un exemple particulier de réalisation de l'invention, la nappe du support est un non-tissé, comportant des fibres hydrosolubles, seules ou en mélange avec des fibres insolubles comme indiqué plus haut, avec au plus de 40% en poids de fibres insolubles par rapport au poids total des fibres constituant la nappe. De préférence, le non-tissé est constitué de fibres hydrosolubles, c'est-à-dire qu'il ne contient pas de fibres insolubles.

Le support peut être sensiblement non rétractable une fois mouillé.

Quand le support ne comporte qu'une seule nappe de fibres, la composition contenant le gélifiant peut être déposée sur les deux faces du support ou sur une seule face, l'autre face du support pouvant alors être utilisée par exemple pour la préhension de l'article.

Quand le support selon la présente invention comporte deux nappes, il peut s'agir notamment de deux nappes de non tissé, tous les modes de réalisation décrits ci-dessous pouvant être utilisés, les nappes pouvant contenir ou non des fibres insolubles, et même une des nappes pouvant être constituée uniquement de fibres insolubles, du moment que l'autre nappe contient des fibres solubles.

Selon un mode particulier de réalisation de l'invention, chacune des nappes est un non-tissé constitué de fibres solubles à une température inférieure ou égale à 30°C, c'est-à-dire que les nappes ne comportent que des fibres hydrosolubles.

Selon un autre mode de réalisation, une des nappes est entièrement soluble dans l'eau et est un non-tissé constitué de fibres solubles à une température inférieure ou égale à 30°C, et l'autre nappe est insoluble et est est un non-tissé constitué de fibres insolubles.

Selon encore un autre mode de réalisation, le support comporte deux nappes contenant des fibres solubles ou partiellement solubles avec au plus 40 % de fibres insolubles, et en outre une nappe constituée de fibres insolubles, constituant un substrat insoluble. Ainsi, le support peut comporter au moins une couche d'un substrat insoluble dans l'eau, c'est-à-dire ne comportant que des fibres insolubles. Dans un exemple particulier de ce mode de réalisation, le support comprend une nappe soluble d'un non-tissé constitué de fibres solubles dans l'eau à une température inférieure ou égale à 30°C, et une nappe insoluble d'un non-tissé constitué de fibres insolubles dans l'eau.

Une structure multicouche avec au moins une couche formée d'un substrat insoluble dans l'eau peut par exemple être utile pour réaliser un article comportant un support en forme de doigt de gant. La couche formée de fibres hydrosolubles est située à l'extérieur de l'article, étant destinée à se solubiliser lors de l'utilisation, après avoir été mouillée ou en venant au contact d'une région mouillée du corps.

Pour fabriquer les nappes en non-tissé, quelles soient solubles ou insolubles, toutes les techniques appropriées de constitution d'un non-tissé à partir de fibres peuvent être utilisées. Par exemple, les fibres peuvent être formées par extrusion et déposées sur un convoyeur pour former une nappe de fibres qui est ensuite consolidée par une technique classique de liage de fibres, telle que par exemple l'aiguilletage, le liage à chaud, le calandrage ou le liage par jets d'air chaud (en anglais *air through bonding*)*,* technique dans laquelle la nappe passe dans un tunnel où est insufflé de l'air chaud. Cette dernière technique est avantageusement utilisée lorsque la nappe est constituée de fibres bicomposant, par exemple des fibres comprenant au moins deux grades d'alcool polyvinylique (PVA), dont les points de fusion ou de ramollissement sont différents, ces fibres étant par exemple co-extrudées de manière à ce que la fibre soit constituée d'au moins un premier grade localisé au coeur de la fibre et d'au moins un deuxième grade localisé en périphérie de la fibre, sous la forme d'une gaine. Le liage des fibres peut être plus facile lorsque la gaine présente un point de fusion plus faible que le coeur.

La nappe de fibres peut encore être formée par cardage de fibres découpées à une longueur de 10 à 50 mm, puis dépôt des fibres sur un convoyeur où la nappe peut ensuite être consolidée par une technique de liage telle que décrite ci-dessus.

Lorsque le support comporte plusieurs couches, que celles-ci soient toutes réalisées avec des fibres hydrosolubles ou non, les différentes couches peuvent être assemblées de multiples manières, par exemple par soudage, collage ou couture, et ces couches peuvent constituer le cas échéant une ou plusieurs cavités contenant une ou plusieurs compositions cosmétiques ou dermatologiques ou plusieurs composants d'une même composition cosmétique à mélanger extemporanément. Lors d'un assemblage par couture, un fil lui-même hydrosoluble peut être utilisé, le cas échéant.

Quand le support comporte plusieurs nappes de non tissé, celles-ci peuvent être assemblées notamment par thermosoudage à leur périphérie de manière à constituer un coussinet capable de retenir dans une cavité intérieure, une composition contenant le gélifiant.

Selon un autre aspect de l'invention, le support est dépourvu d'adhésif, notamment d'adhésif sensible à la pression.

La densité du support pourra dépendre des applications. Le support peut présenter, par exemple, une densité inférieure ou égale à 0,1 g/cm ou bien supérieure à 0,1 g/ cm³. Selon un mode préféré de réalisation de l'invention, le support a une densité inférieure ou égale à 0,1 g/cm³, mieux allant de 0,01 g/cm³ à 0,1 g/cm³, ce qui permet d'avoir un support très aéré, qui, de ce fait, se dissout dans l'eau plus facilement.

La composition contenant au moins un gélifiant hydrosoluble représente entre 10 et 1000 % en poids par rapport au poids du support, et de préférence entre 10 et 500 % en poids par rapport au poids du support en entendant ici par «poids du support», le poids du support seul, sans le poids de la composition contenant le gélifiant hydrosoluble. Si la composition ne contient que le gélifiant hydrosoluble, c'est celui-ci qui peut représenter entre 10 et 1000 % en poids par rapport au poids du support, et de préférence entre 10 et 500 % en poids par rapport au poids du support.

### Gélifiants hydrosolubles

La composition portée par le support contient un ou plusieurs gélifiants hydrosolubles qui gonflent en moins de 30 secondes dans l'eau à une température de 20°C à 30°C. Les gélifiants contenus dans la composition portée par le support peuvent être choisis notamment parmi les polysaccharides qui gonflent en moins de 30 secondes dans l'eau à une température de 20°C à 30°C, notamment les gommes qui gonflent en moins de 30 secondes dans l'eau à une température de 20°C à 30°C, les amidons modifiés et leurs mélanges.

Par « hydrosolubles qui gonflent en moins de 30 secondes dans l'eau à une température de 20°C à 30°C», on entend des gélifiants qui se dissolvent ou gonflent facilement dans l'eau à une température inférieure à 30°C, allant notamment de 20° à 30°C, et à une concentration de 5 % à 50 % en poids, pour donner un gel visqueux qui ne coule pas. Ces gélifiants sont aptes à leur état sec à absorber spontanément au moins 10 fois leur poids d'eau , notamment d'eau distillée, « spontanément » signifiant sans agitation et sans chauffage, c'est-à-dire à la température ambiante (20 à 25°C).

Comme gélifiants hydrosolubles, on peut citer par exemple la gomme de karaya, la gomme de konjac, les amidons modifiés, et leurs mélanges.

Comme amidons modifiés, ne conviennent que ceux qui gonflent spontanément dans l'eau, ce qui n'est pas le cas par exemple de l'amidon modifié par un dérivé de méthylolurée ou par l'épichlorhydrine ou par l'anhydride octénylsuccinique tel que celui commercialisé sous la dénomination Dry Flo qui est hydrophobe et n'est pas approprié dans la présente invention. Ces amidons ne gonflent pas dans l'eau et ne répondent donc pas à la définition du gélifiant revendiqué dans la présente invention.

Parmi les amidons modifiés convenant à la présente invention, on peut citer par exemple :
- les hydroxyalkyl-C2-C5-amidons, en particuler les amidons hydroxyalkylpropylés, notamment les phosphates d'amidon hydroxypropylés comme ceux commercialisés sous les dénominations Structure XL et Structure Zea (hydroxypropyl Starch Phosphate) par la société National Starch ;
- les amidons prégélatinisés, notamment ceux respectivement de mais et de pomme de terre commercialisés sous les dénominations Novation 5600 et 6600, par la société National Starch ;
- les amidons modifiés de maïs, commercialisés sous les dénominations Ultra-tex 1, 2, HV, 2000, par la société National Starch ;
- les amidons greffés sur des polymères ou copolymères, tels que les amidons greffés sur du polyacrylate de sodium, commercialisés sous les dénominations Sanfresh ST-100C, ST100MC, IM-300MC (nom INCI Sodium polyacrylate Starch) par la société Sanyo Chemical Industries ; et les amidons greffés sur un copolymère d'acrylamide, comme ceux commercialisés sous les dénominations Water Lock A-240, A-180, B-204, D-223, A-100, C-200, D-223, G-400 par la société Grain Processing (nom INCI: Starch/acrylamide/sodium acrylate copolymer),
- et leurs mélanges.

L'amidon modifié est de préférence choisi parmi les hydroxyalkyl-C2-C5-amidons, les amidons prégélatinisés, les amidons greffés sur un polymère ou un copolymère, en particulier les amidons greffés sur du polyacrylate de sodium, les amidons greffés sur un copolymère d'acrylamide, et leurs mélanges.

La quantité de gélifiant hydrosoluble dans la composition peut aller par exemple de 5 à 100 % en poids, mieux de 10 à 100 % en poids, et encore mieux de 20 à 100 % en poids par rapport au poids total de la composition.

### Compositions

Les compositions portées par le support et contenant le ou les gélifiants hydrosolubles sont des compositions anhydres. Elles sont de préférence sous forme pulvérulente ou pâteuse, et plus préférentiellement sous forme pulvérulente. Ce sont des compositions adaptées à une application topique, notamment des compositions cosmétiques ou dermatologiques.

Ainsi, les compositions cosmétiques utilisables dans l'invention peuvent être par exemple :
- des émulsions lyophilisées ou atomisées, telles que celles décrites dans le document FR-A-2,727,312 ou celles à base d'amidon modifié décrites dans le document EP-A-0 938 892. Ces émulsions sont obtenues par lyophilisation ou atomisation d'une émulsion H/E contenant une phase pulvérulente, conduisant à des laits ou des crèmes par mélange avec l'eau lors de leur utilisation.
- des compositions moussantes sous forme de poudres, contenant des tensioactifs pulvérulents, comme celles à base d'amidon, décrites dans le document EP-A-0 925 777, conduisant à de la mousse par mélange avec l'eau lors de leur utilisation.
- des compositions exfoliantes contenant des scrubs ou particules exfoliantes.
- des compositions formées par simple mélange des constituants, ceux-ci étant de préférence sous forme de poudres.

La composition peut éventuellement contenir une certaine quantité d'eau au moment de son imprégnation sur le support. Toutefois, de manière à éviter sa solubilisation prématurée, l'eau introduite sur le support lors de son imprégnation doit être éliminée par les moyens classiquement utilisés pour la déshydratation des compositions contenant de l'eau, comme par exemple le chauffage. Cependant, la composition peut contenir une certaine quantité d'eau qui est généralement de l'eau liée et qui peut provenir notamment des matières premières hygroscopiques qui contiennent de l'eau, telles que les amidons La quantité d'eau finale dans la composition présente sur l'article est d'au maximum 20 % en poids et de préférence au maximum 10 % en poids par rapport au poids total de la composition.

Dans le cas d'une composition colorée, l'article peut être conditionné dans un emballage comportant, le cas échéant, un témoin coloré représentatif de la couleur de la composition obtenue après dissolution de l'article, ceci afin de renseigner le consommateur avant l'achat.

Lorsque la composition doit être déposée sur le support par l'utilisateur lui-même, la composition et le support peuvent être proposés sous la forme d'un kit, par exemple. La composition est par exemple livrée en une quantité suffisante pour permettre d'en distribuer une pluralité de doses sur un ensemble de supports destinés à être utilisés successivement.

### Additifs

La composition portée par le support peut contenir, selon l'utilisation finale de l'article, d'autres composés que les gélifiants hydrosolubles.

Ces additifs peuvent être notamment anhydres ou sous forme solide (poudre). Ils peuvent être notamment choisis parmi ceux généralement utilisés dans les domaines cosmétique et dermatologique, tels que, par exemple, les tensioactifs, les séquestrants, les parfums, les antioxydants, les actifs, les conservateurs, les matières colorantes (comme les pigments et les colorants hydrophiles) et les charges minérales et/ou les charges organiques telles que les particules exfoliantes et le kaolin. Ces adjuvants ainsi que leurs quantités doivent être tels qu'ils ne modifient pas la propriété recherchée pour la composition de l'invention. Selon un mode particulier de réalisation de l'invention, ces additifs peuvent être encapsulés ou adsorbés sur des poudres.

Les tensioactifs peuvent être en particulier des tensioactifs moussants. Comme tensioactifs moussants, on peut utiliser tous ceux habituellement utilisés dans le domaine cosmétique, ces tensioactifs pouvant être anioniques, non ioniques, cationiques, amphotères ou zwitterioniques.

La quantité de tensioactif(s) moussant(s) peut aller par exemple de 2 à 80 % en poids, de préférence de 10 à 70 % en poids, et mieux de 10 à 50 ù en poids par rapport au poids total de la composition.

Comme tensioactifs anioniques moussants, on peut citer par exemple les sels d'acide gras qui constituent les savons et qui sont dérivés d'un acide gras ayant une chaîne alkyle comportant de 6 à 22 atomes de carbone, de préférence de 8 à 18 atomes de carbone ; les alkylsulfates et alkylethersulfates ; les sulfonates,; les sels alcalins de N-acylaminoacides tels que les sarcosinates, les alaninates, les glutamates, les aspartates, les glycinates ; et leurs mélanges.

Comme tensioactifs non ioniques, on peut citer par exemple les esters de sucre, les éthers de sucre comme les alkyl polyglucosides (APG), les condensats d'oxydes d'alkylène et d'alkyl phénols, les éthers d'alcool gras et de polyols, et leurs mélanges.

Comme tensioactifs amphotères ou zwitterioniques, on peut citer les bétaïnes et leurs dérivés, les sultaïnes et leurs dérivés, les dérivés d'imidazolinium, et leurs mélanges.

Les tensioactifs préférés sont ceux en poudre, tels que par exemple le lauryl sulfate de sodium comme le produit commercialisé sous la dénomination Empicol LZ D par la société Allbright & Wilson ou sous la dénomination Tensopol USP97 par la société Tensachem ; la cocamidopropylbetaine comme le produit commercialisé sous la dénomination Tegobetain CK D par la société Degussa ; le lauroyl glutamate de sodium comme le produit commercialisé sous la dénomination Amisoft LS 11 par la société Ajinomoto ; le myristoyl glutamate monosodique comme le produit commercialisé sous la dénomination Acylglutamate MS 11 par la société Ajinomoto ; le mélange de laureth sulfate de sodium et de silice, commercialisé sous la dénomination Texapon KE 2713 par la société Cognis ; le disodium cocamido MEA-sulfosuccinate comme le produit commercialisé sous la dénomination Mackanate CM 100 par la société Mac Intyre ; le methyl cocoyl taurate de sodium, comme le produit commercialisé sous la dénomination Tauranol WSP par la société Finetex ; le decyl d-galactoside uronate de sodium comme le produit commercialisé sous la dénomination Decyl d-galactoside uronate de sodium par la société Ard-Soliance ; le lauroyl methyl beta-alanine (forme acide) commercialisé sous la dénomination LMA-H par la société Mitsui Toatsu ; la n-lauroyl-n-hydroxyethyl-beta-alanine commercialisée sous la dénomination LHEA par la société Mitsui Toatsu ; le cocoyl glycinate de sodium commercialisé sous la dénomination Amilite GCS-11(F) par la société Ajinomoto ; le cocoyl isethionate de sodium comme le produit commercialisé sous la dénomination Jordapon CI P par la société BASF ; le lauryl sulfoacétate de sodium, comme le produit commercialisé sous la dénomination Lathanol LAL poudre par la société Stepan ; le myristate de potassium comme le produit commercialisé sous la dénomination Myristate de potassium (DUB MK) par la société Stearinerie Dubois ; le laurate de potassium comme le produit commercialisé sous la dénomination Laurate de potassium (DUB LK) par la société Stearinerie Dubois, et le laurate de sucrose comme le produit commercialisé sous la dénomination Grilloten LSE 87 par la société Degussa.
Les actifs peuvent être choisis notamment parmi les agents kératolytiques, les hydratants, les apaisants et les antimicrobiens.

Comme hydratants, on peut citer les polyols tels que la glycérine ; les composés agissant sur la fonction barrière, en vue de maintenir l'hydratation du stratum corneum, ou les composés occlusifs, en particulier les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline ; les composés augmentant directement la teneur en eau du stratum corneum, tel que le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, et la N-α-benzoyl-L-arginine ; et leurs mélanges.

Comme agents kératolytiques, on peut citer les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique, et leurs mélanges.

Comme agents apaisants utilisables dans la composition selon l'invention, on peut citer par exemple les triterpènes pentacycliques et les extraits de plantes (ex : Glycyrrhiza glabra) en contenant comme l'acide β-glycyrrhétinique et ses sels et/ou ses dérivés (l'acide glycyrrhétinique monoglucuronide, le stearyl glycyrrhetinate, l'acide 3- stéaroyloxy glycyrrhetique), l'acide ursolique et ses sels, l'acide oléanolique et ses sels, l'acide bétulinique et ses sels, les extraits de plantes telles que *Paeonia suffruticosa* et / ou *lactiflora, Laminaria saccharina, Boswellia serrata, Centipeda cunnighami, Helianthus annuus, Linum usitatissimum, Cola nitida, Epilobium Angustifolium, Aloe vera, Bacopa monieri,* les sels de l'acide salicylique et en particulier le salicylate de zinc, l'huile de Canola, le bisabolol et les extraits de camomille, l'allantoïne, le Sépivital EPC (diesterphosphorique de vitamine E et C) de Seppic, les huiles insaturées en oméga 3 telles que les huiles de rosier muscat, de cassis, d'ecchium, ou de poisson, des extraits de plancton, la capryloyl glycine, le Seppicalm VG (sodium palmitoylproline et nymphea alba) de Seppic, les tocotrienols, le piperonal, un extrait de clou de girofle, les phytostérols, la cortisone, l'hydrocortisone, l'indométhacine et la beta méthasone.

Comme antimicrobiens, on peut citer par exemple le 2,4,4'-trichloro-2'-hydroxy diphényl éther (ou triclosan), le 3,4,4'-trichlorocarbanilide (ou triclocarban) le phénoxyéthanol, le phénoxypropanol, le phénoxyisopropanol, l'hexamidine iséthionate, le métronidazole et ses sels, le miconazole et ses sels, l'itraconazole, le terconazole, l'éconazole, le ketoconazole, le saperconazole, le fluconazole, le clotrimazole, le butoconazole, l'oxiconazole, le sulfaconazole, le sulconazole, le terbinafine, le ciclopiroxe, le ciclopiroxolamine, l'acide undécylenique et ses sels, le peroxyde de benzoyle, l'acide 3-hydroxy benzoïque, l'acide 4-hydroxy benzoïque, l'acide phytique, l'acide N-acétyl-L-cystéine, l'acide lipoïque, l'acide azélaïque et ses sels, l'acide arachidonique, le résorcinol, l'octopirox, l'octoxyglycérine, l'octanoylglycine, le caprylyl glycol, l'acide 10-hydroxy-2-décanoïque, le dichlorophenyl imidazol dioxolan et ses dérivés décrits dans le brevet WO-A-93/18743, le farnesol, les phytosphingosines et leurs mélanges.

Comme vitamines, on peut utiliser les vitamines ou provitamines hydrosolubles ou liposolubles, comme par exemple les vitamines A (rétinol), C (acide ascorbique), B3 ou PP (niacinamide), B5 (panthénol), B6 ou pyridoxine, E (tocophérol), K1, le bêta-carotène, et les dérivés de ces vitamines et notamment leurs esters, et leurs mélanges.

La composition peut contenir aussi des exfoliants, notamment pour constituer une composition de gommage ou un scrub pour le visage ou le corps. Comme exfoliants, on peut citer par exemple des particules exfoliantes ou gommantes d'origines minérale, végétale ou organique.

La composition selon l'invention peut contenir aussi une ou plusieurs composés lipophiles, corps gras et notamment huiles, ou actif huileux, dans la mesure où ces composés lipophiles ne perturbent pas le gonflement du gélifiant. S'ils sont présents, les corps gras peuvent constituer jusqu'à 50 % de la composition portée par le support, par exemple de 0 à 50 % en poids, de préférence de 0,1 à 40 % en poids, mieux de 0,1 à 20 % en poids et encore mieux de 0,5 à 10 % en poids par rapport au poids total de la composition. Ces composés lipophiles peuvent contribuer à avoir un article mieux rinçable.

Bien entendu, l'homme de l'art veillera à choisir ce ou ces éventuels additifs et/ou leurs quantités de manière telle que les propriétés avantageuses attachées intrinsèquement à la composition conforme à l'invention ne soient pas, ou substantiellement pas, altérées par la ou les adjonctions envisagées.

L'article selon l'invention constitue notamment un patch, et il peut être utilisé comme composition pour le nettoyage et le démaquillage de la peau, pour le traitement des signes de l'âge, pour le traitement des peaux grasses, pour l'hydratation de la peau, pour le gommage de la peau, pour la protection solaire et le traitement après-soleil, pour le traitement des peaux sensibles ou sensibilisées..

L'article selon l'invention peut constituer notamment un patch à rincer ou un article-deux-en-un à rincer pour le traitement spécifique d'une zone retreinte ou comme produit unidose à appliquer sur une plus large zone en ayant préalablement humidifié le produit.

Il est utilisé généralement en humidifiant la zone à traiter et en y appliquant le patch qui se transforme en gel, puis en l'éliminant par simple rinçage.

L'article selon l'invention peut trouver des applications dans le soin en général, par exemple pour le nettoyage et le démaquillage de la peau, pour le traitement des signes de l'âge, pour le traitement des peaux grasses, pour l'hydratation de la peau, pour le gommage de la peau (y compris comme peelings), pour la protection solaire et le traitement après-soleil, ainsi que pour le traitement des peaux sensibles ou sensibilisées.

L'article selon l'invention peut constituer notamment un produit de nettoyage de la peau, un produit exfoliant, un produit de soin de la peau.

Les exemples qui suivent servent à illustrer l'invention sans toutefois présenter un caractère limitatif. Les quantités indiquées sont en % en poids sauf mention contraire, et elles correspondent, sauf mention contraire, à la quantité de matière première et non à la quantité de matière active. Les noms des composés utilisés sont indiqués en nom INCI, en nom chimique ou en nom commercial.

### EXEMPLES

L'article utilisé dans les exemples est réalisé avec un support en fibres Kuralon KIl WN2 à base de PVA. Il est obtenu en thermosoudant à leur périphérie deux couches de grammage 80 g/m². L'article se présente sous la forme d'un disque de 3 cm de diamètre, comportant une cavité dans laquelle est introduite la composition en une quantité d'environ 0,3 grammes

| | Exemple 1 selon l'invention | Exemple comparatif | Exemple 2 selon l'invention | Exemple 3 selon l'invention |
|---|---|---|---|---|
| Amidon modifié (1) | 100 | - | 80 | - |
| Amidon greffé polyacrylate (2) | - | - | - | 69 |
| Polymère d'AMPS | - | 100 | - | - |
| Acide glycolique | - | - | 20 | - |
| Kaolin | - | - | - | 10 |
| Triclosan | - | - | - | 1 |
| Lauroyl Glutamate de sodium (4) | - | - | | 20 |

| | | | | |
|---|---|---|---|---|
| (1) Structure XL (National Starch) (2) Sanfresh ST-100C (Sanyo Chemical Industries) (3) Hostacerin AMPS (Clariant) (4) Aminosoft LS11 (Ajinomoto) | | | | |

Les exemples ont été obtenus en mélangeant les poudres, puis en introduisant environ 0,3 grammes du mélange dans la cavité du support, qui a été ensuite fermée par soudage.

Pour tous les exemples, l'article est préalablement mouillé avec une quantité d'eau supérieure ou égale à 2 ml avant d'être appliquée sur la zone à traiter. Dans les 3 exemples selon l'invention, on obtient un gel en moins de 30 secondes, alors que l'exemple comparatif donne une solution fluide.

Le patch de l'exemple 2 constitue un peeling : après addition d'environ 2 ml d'eau, on l'applique sur la peau, puis que l'on rince après un temps de pose d'environ 5 minutes.

Le patch de l'exemple 3 constitue un soin pour peaux grasses à rincer après application. Il peut être utilisé en soin concentré à appliquer sur la zone du nez pendant 5 minutes après l'avoir réhydraté avec environ 2ml d'eau, ou en produit de nettoyage quotidien après dilution avec une plus grande quantité d'eau (plus de 4 ml d'eau).

## Revendications

1. Article cosmétique ou dermatologique comportant :
- un support sous forme d'au moins une nappe comprenant des fibres solubles dans l'eau à une température inférieure ou égale à 30°C, et
- une composition portée par le support, contenant au moins un gélifiant hydrosoluble qui gonfle en moins de 30 secondes dans l'eau à une température de 20 °C à 30 °C.

2. Article selon la revendication 1, **caractérisé en ce que** les fibres solubles dans l'eau à une température inférieure ou égale à 30°C sont réalisées avec de l'alcool polyvinylique.

3. Article selon la revendication 1 ou 2, **caractérisé en ce que** la nappe de fibres est un non-tissé.

4. Article selon l'une quelconque des revendications précédentes, **caractérisé en ce que** la nappe de fibres comprend des fibres insolubles dans l'eau.

5. Article selon la revendication précédente, **caractérisé en ce que** la quantité de fibres insolubles dans l'eau est d'au plus 40 % en poids par rapport au poids total des fibres du support.

6. Article selon l'une quelconque des revendications précédentes, **caractérisé en ce que** le support comporte au moins deux nappes dont au moins une contient des fibres solubles dans l'eau à une température inférieure ou égale à 30°C.

7. Article selon l'une quelconque des revendications précédentes, **caractérisé en ce qu'**il comporte :
- au moins deux nappes définissant entre elles une cavité, l'une au moins des nappes comprenant des fibres solubles dans l'eau à une température inférieure ou égale à 30 °C,
- la cavité contenant une composition comportant un gélifiant hydrosoluble qui gonfle en moins de 30 secondes dans l'eau à une température de 20°C à 30°C.

8. Article selon la revendication précédente, **caractérisé en ce que** les deux nappes de fibres sont des non-tissés.

9. Article selon la revendication 6, **caractérisé en ce que** l'une des nappes est un non-tissé constitué de fibres solubles dans l'eau à une température inférieure ou égale à 30°C, et l'autre nappe est un non-tissé constitué de fibres insolubles dans l'eau.

10. Article selon l'une quelconque des revendications 6 à 8, **caractérisé en ce que** les deux nappes sont assemblées à leur périphérie.

11. Article selon la revendication précédente, **caractérisé en ce que** les nappes sont thermosoudées.

12. Article selon l'une quelconque des revendications 1 à 3, 6 ou 7, **caractérisé en ce que** le support est entièrement soluble dans l'eau.

13. Article selon l'une quelconque des revendications précédentes, **caractérisé en ce que** le gélifiant est apte à son état sec à absorber spontanément au moins 10 fois son poids d'eau.

14. Article selon l'une quelconque des revendications précédentes, **caractérisé en ce que** la quantité de gélifiant hydrosoluble va de 5 à 100 % en poids par rapport au poids total de la composition.

15. Article selon l'une quelconque des revendications précédentes, **caractérisé en ce que** le gélifiant est choisi parmi la gomme de karaya, la gomme de konjac, les amidons modifiés, et leurs mélanges.

16. Article selon la revendication précédente, **caractérisé en ce que** l'amidon modifié est chois parmi les hydroxyalkyl-C2-C5-amidons, les amidons prégélatinisés, les amidons greffés sur un polymère ou un copolymère, et leurs mélanges.

17. Article selon l'une quelconque des revendications précédentes, **caractérisé en ce que** la composition portée sur le support représente entre 10 et 1000 % en poids, par rapport au poids du support.

18. Article selon l'une quelconque des revendications précédentes, **caractérisé en ce que** la composition portée par le support contient en outre au moins un composé choisi parmi les tensioactifs moussants, les polymères, les composés lipophiles, les exfoliants, les actifs, et leurs mélanges.

19. Article selon l'une quelconque des revendications précédentes, **caractérisé en ce qu'**il constitue une composition pour le nettoyage et le démaquillage de la peau, pour le traitement des signes de l'âge, pour le traitement des peaux grasses, pour l'hydratation de la peau, pour le gommage de la peau, pour la protection solaire et le traitement après-soleil, pour le traitement des peaux sensibles ou sensibilisées.

20. Article selon l'une quelconque des revendications précédentes, **caractérisé en ce qu'**il constitue un patch.

21. Procédé de traitement cosmétique d'une matière kératinique, comportant :
- la formation d'une composition par dissolution dans l'eau, d'un support sous forme d'au moins une nappe comprenant des fibres solubles dans l'eau à une température inférieure ou égale à 30°C, le dit support portant au moins un gélifiant hydrosoluble qui gonfle en moins de 30 secondes dans l'eau à une température de 20°C à 30°C;
- l'application de la composition ainsi formée sur la matière kératinique.

22. Ensemble comportant :
- un emballage,
- au moins un article selon l'une quelconque des revendications 1 à 19.
